(19) Europäisches Patentamt

European Patent Office

Office européen des brevets

(11) EP 2 322 111 B1

(12) EUROPEAN PATENT SPECIFICATION

(45) Date of publication and mention of the grant of the patent:
06.04.2016 Bulletin 2016/14

(51) Int Cl.:
A61B 18/14 (2006.01)     A61B 18/12 (2006.01)
A61B 18/00 (2006.01)

(21) Application number: 10191321.8

(22) Date of filing: 16.11.2010

(54) **Multi-phase electrode**

Multiphasenelektode

Électrode multiphase

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.11.2009 US 619462**

(43) Date of publication of application:
**18.05.2011 Bulletin 2011/20**

(60) Divisional application:
**16156267.3**

(73) Proprietor: **Covidien LP
Mansfield, MA 02048 (US)**

(72) Inventor: **Ladtkow, Casey M.
Westminister, CO 80031 (US)**

(74) Representative: **Soames, Candida Jane et al
Maschio & Soames IP Limited
20 Carlton Crescent
Southampton, SO15 2ET (GB)**

(56) References cited:
**WO-A2-96/18349          WO-A2-2005/048862
US-A- 5 935 159          US-A1- 2001 008 967
US-A1- 2008 281 322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

*Technical Field*

The present disclosure relates to electrosurgical apparatuses, systems and methods. More particularly, the present disclosure is directed to an electrosurgical instrument having a plurality of electrodes that operates at different phases.

*Background of Related Art*

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current to a surgical site to cut, ablate, coagulate or seal tissue.

In monopolar electrosurgery, the active electrode is typically a part of the surgical instrument held by the surgeon that is applied to the tissue to be treated. A patient return electrode is placed remotely from the active electrode to carry the current back to the generator and safely disperse current applied by the active electrode. The return electrodes usually have a large patient contact surface area to minimize heating at that site. Heating is caused by high current densities which directly depend on the surface area. A larger surface contact area results in lower localized heat intensity. Return electrodes are typically sized based on assumptions of the maximum current utilized during a particular surgical procedure and the duty cycle (i.e., the percentage of time the generator is on).

US 2001/008967 discloses an ablation apparatus. The apparatus has a catheter comprising a distal segment having an electrode device and a handle located outside the patient. A preferred embodiment of the electrode device includes twelve band electrodes arranged spaced apart in a substantially linear array along the distal segment of the catheter. The electrodes are coupled to a power source by leads within the catheter.

US 5935159 discloses a defibrillator having defibrillator electrodes disposed on a a lead. The lead has an insulative outer sheath containing a core. The core has grooves in the surface thereof. The grooves contain insulated conductors which connect the electrodes to a source of defibrillation voltage.

## SUMMARY

According to the present invention there is provided an electrosurgical instrument comprising: a core including an elongated body portion, the core including a plurality of channels defined therein that are disposed spaced apart on an outer surface of the core ; a plurality of electrodes disposed about the core, wherein each of the plurality of electrodes has a tubular shape , the electrodes being spaced apart by one or more dielectric spacers the plurality of the electrodes being couplable to an electrosurgical generator configured to supply phased electrosurgical voltage to the plurality of electrodes to generate a potential difference between at least two of the plurality of the electrodes; and a plurality of conductors , each formed from a spring member, configured to couple respective ones of the plurality of electrodes to the electrosurgical generator, each of the plurality of conductors being disposed within a respective one of the plurality of channels

The core may include a tapered tip) at a distal end thereof configured to penetrate tissue.

A distal most electrode of the plurality of electrodes may include a tapered tip at a distal end thereof configured to penetrate tissue.

The core may include a lumen defined therethrough. The electrosurgical instrument may further comprise: an outer tube disposed within the lumen; and an inner tube disposed concentrically relative to the outer tube, wherein the outer tube and the inner tube are coupled to a coolant system configured to circulate a coolant therethrough.

The at least one dielectric spacer may be formed integrally with the core.

The conductors may be separated by the core and only contact the corresponding electrodes, and wherein the core has a dielectric structure so that the dielectric structure of the core obviates the need for an insulating sheath about each conductor.

The electrosurgical instrument, in use, is coupled to an electrosurgical generator (20) coupled to each of the plurality of the electrodes and configured to supply phased electrosurgical voltage to the plurality of electrodes to generate a potential difference between at least two of the plurality of the electrodes.

The electrosurgical instrument may be connected to a coolant system including a supply tank configured to store a coolant; and a supply pump coupled to the supply tank and to the electrosurgical instrument and configured to circulate a coolant therethrough.

## BRIEF DESCRIPTION OF THE DRAWINGS

Various embodiments of the present disclosure are described herein with reference to the drawings wherein:

Fig. 1 is a schematic diagram of an electrosurgical system ;

Fig. 2 is a schematic block diagram of the electrosurgical generator of Fig.1;

Fig. 3 is perspective, cross-sectional view of an electrode assembly ;

Fig. 4 is a side, cross-sectional view of an electrode assembly of Fig. 3;

Fig. 5 is a side, cross-sectional view of a conductor according to one embodiment of the present disclosure;

Fig. 6 is perspective, cross-sectional view of another electrode assembly;

Fig. 7 is a side, cross-sectional view of an electrode assembly of Fig. 6; and

Fig. 8 is a side, cross-sectional view of another electrode assembly.

## DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

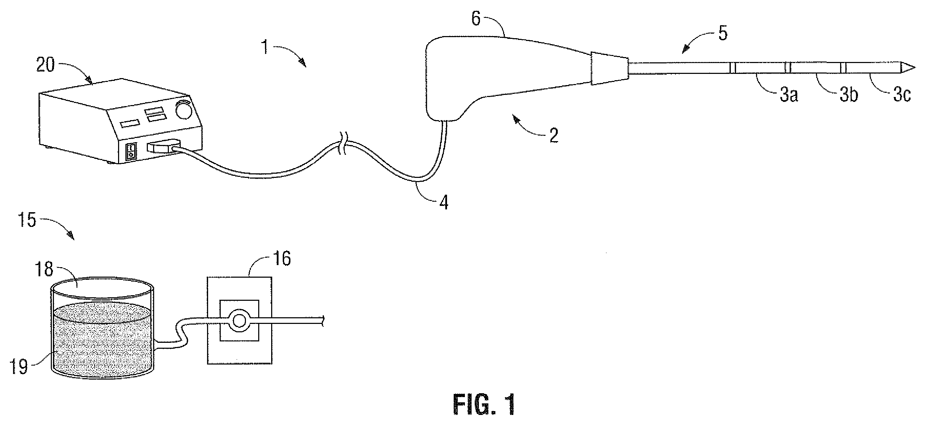

Fig. 1 is a schematic illustration of a multipolar electrosurgical system 1 according to one embodiment of the present disclosure. The system includes one or more multipolar electrosurgical instruments 2 having a housing 6 with an electrode assembly 5 coupled thereto and extending distally therefrom. The electrode assembly 5 includes one or more electrodes 3a, 3b, 3c, etc. (e.g., electrosurgical cutting probe, ablation electrode(s), etc.) for treating tissue of a patient. The instrument 2 is coupled to a generator 20 via a cable 4 that encloses a plurality of supply and return lines. More specifically, the electrosurgical RF energy is supplied to the instrument 2 is via the generator 20, allowing the instrument 2 to coagulate, ablate and/or otherwise treat tissue. The energy is also returned to the generator 20 through the electrodes 3a, 3b, 3c, etc.

The instrument 2 may also be coupled to a coolant system 15 that includes a supply pump 16 and to a supply tank 18. The supply pump 16 may be a peristaltic pump or any other suitable type of pump. The supply tank 18 stores the coolant fluid 19 and, in one embodiment, may maintain the fluid at a predetermined temperature. In another embodiment, the coolant fluid 19 may be a gas and/or a mixture of fluid and gas. The coolant system 15 is configured to circulate the coolant fluid 19 through the electrode assembly 5, thereby cooling the electrodes as discussed in more detail below.

The generator 20 is configured to operate in a variety of modes. In one embodiment, the generator 20 may operate in the following modes: cut, blend, division with hemostasis, fulgurate and spray. Each of the modes operates based on a preprogrammed power curve that dictates how much power is outputted by the generator 20 at varying impedance ranges of the load (e.g., tissue).

Each of the power curves includes a constant power, constant voltage and constant current ranges that are defined by the user-selected power setting and the measured minimum impedance of the load.

In a cut mode, the generator 20 supplies a continuous sine wave at a predetermined frequency (e.g., 472 kHz) having a crest factor of 1.5 or less in the impedance range of 100Ω to 2,000Ω. The cut mode power curve may include three regions: constant current into low impedance, constant power into medium impedance and constant voltage into high impedance. In the blend mode, the generator supplies bursts of a sine wave at the predetermined frequency, with the bursts reoccurring at a first predetermined rate (e.g., about 26.21 kHz). In one embodiment, the duty cycle of the bursts may be about 50%. The crest factor of one period of the sine wave may be iess than 1.5. The crest factor of the burst may be about 2.7.

The division with hemostasis mode includes bursts of sine waves at a predetermined frequency (e.g., 472 kHz) reoccurring at a second predetermined rate (e.g., about 28.3 kHz). The duty cycle of the bursts may be 25%. The crest factor of one burst may be 4.3 across an impedance range of 100Ω to 2,000Ω. The fulgurate mode includes bursts of sine waves at a predetermined frequency (e.g., 472 kHz) reoccurring at a third predetermined rate (e.g., about 30.66 kHz). The duty cycle of the bursts may be 6.5% and the crest factor of one burst is 5.55 across an impedance range or 100Ω to 2,000Ω. The spray mode will be bursts of sine waves at a predetermined frequency (e.g., 472 kHz) reoccurring at a fourth predetermined rate (e.g., about 21.7 kHz). The duty cycle of the bursts may be 4.6% and the crest factor of one burst may be 6.6 across the impedance range of 100Ω to 2,000Ω.

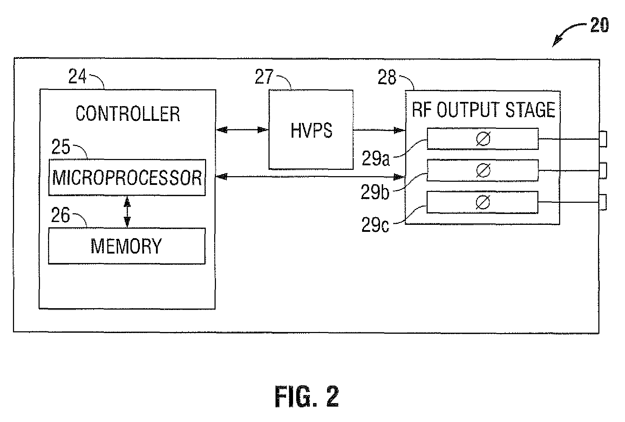

Fig. 2 shows a schematic block diagram of the generator 20 having a controller 24, a high voltage DC power supply 27 ("HVPS") and an RF output stage 28. The HVPS 27 is connected to an AC source (e.g., electrical wall outlet) and provides high voltage DC power to an RF output stage 28, which then converts high voltage DC power into RF energy and delivers the RF energy to the instrument 2. In particular, the RF output stage 28 generates sinusoidal waveforms of high RF energy. The RF output stage 28 is configured to operate in a plurality of modes, during which the generator 20 outputs corresponding waveforms having specific duty cycles, peak voltages, crest factors, etc.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop, in which a plurality of sensors measure a variety of tissue and energy properties (e.g., tissue impedance, tissue temperature, output current and/or voltage, etc.), and provide feedback to the controller 24.

Such sensors are within the purview of those skilled in the art. The controller 24 then signals the HVPS 27 and/or RF output stage 28, which then adjusts the DC and/or RF power supply, respectively. The controller 24 also receives input signals from the input controls of the generator 20 and/or the instrument 2a. The controller 24 utilizes the input signals to adjust power outputted by the generator 20 and/or performs other control functions thereon.

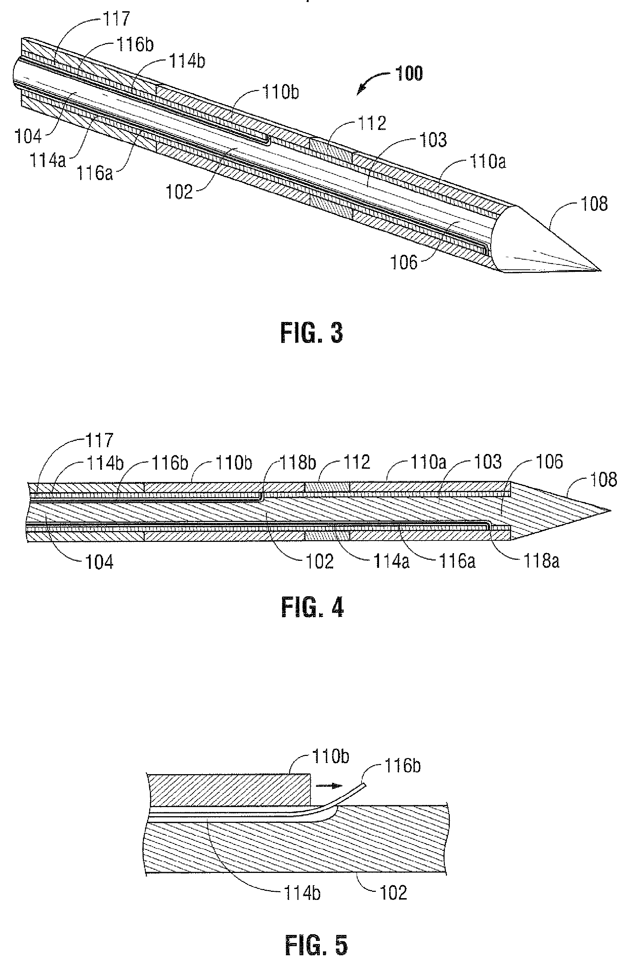

Figs. 3 and 4 show an electrode assembly 100 according to an embodiment of the present disclosure. The electrode assembly 100 includes a core 102 having an elongated body portion 103. The body portion 103 includes a proximal end 104 coupled to the housing 6 (Fig. 1) and a distal end 106 having a tip 108. The tip 108 may be formed integrally with the body portion 103. The tip 108 includes a tapered portion terminating in a sharp tip to allow for insertion into tissue with minimal resistance. In those cases where the energy applicator is inserted into a pre-existing opening, the tip 108 may be rounded or flat.

The electrode assembly 100 also includes two or more electrodes 110a and 110b. The electrodes 110a and 110b have a substantially cylindrical tubular shape and are disposed about the core 102. The electrodes 110a and 110b are separated by a dielectric spacer 112 that also has a cylindrical tubular shape. The electrodes 110a and 110b may be formed from any suitable medical grade conductor suitable for contacting tissue (e.g., stainless steel).

In one embodiment, the dielectric spacer 112 may be integrally formed with the core 102. The core 102, the dielectric spacer 112 and the tip 108 may be formed from a suitable polymeric material, which may include, for example, thermoplastics including reinforced or unreinforced polymers, e.g., polyamide (nylon) or polyaramid (e.g., KEVLAR® manufactured by E. I. du Pont de Nemours and Company of Wilmington, Delaware, United States), or any suitable polymeric composite, e.g., polymers filled with carbon particles, silica, conductive particles such as metal particles or conductive polymers, or combinations thereof.

The core 102 includes a plurality of channels 114a and 114b defined therein. In this embodiment, the conductors 116a and 116b include an insulating sheath to prevent short-circuiting. In another embodiment, the channels 114a and 114b house one or more conductors 116a and 116b, respectively. The conductors 116a and 116b may be encased in an insulating sheath (not shown) and coupled to the electrodes 110a and 110b, respectively. The channels 114a and 114b are disposed apart (e.g., radially) within the core 102 and extend up to the respective electrodes 110a and 110b. The dielectric structure of the core 102 obviates the need for providing an insulating sheath about the conductors 116a and 116b since the conductors 116a and 116b are separated by the core 102 and only contact the corresponding electrodes 110a and 110b. The core 102 may also be formed from conductive materials (e.g., metal) and may include a sheath 117 disposed over the outer surface thereof to insulate the core 102 from the electrodes 110a and 110b.

The channels 114a and 114b are disposed on an outer surface of the core 102. As shown in Fig. 4, the core 102 may include a sheath 117 disposed over the body portion 103 to insulate the conductors 116a and 116b. The sheath 117 includes openings 118a and 118b to provide for coupling of the conductors 116a and 116b to the electrodes 114a and 114b (Fig. 4).

As shown in Fig. 5, the conductors 116a and 116b are formed from a spring member (e.g., hardened spring steel) to ensure that the conductors 116a and 116b are pushed into the corresponding channels 114a and 114b as the electrodes 110a and 110b are pushed into place. This configuration also secured the conductors 116a and 116b within the channels 114a and 114b.

With reference to Fig. 2, in one embodiment, the generator 20 is a multi-phase radio-frequency energy generator that can supply electrosurgical energy across each voltage line corresponding to a specific phase. Each of the electrodes 110a and 110b is connected to a voltage line of the generator 20. The generator includes a plurality of phase-shifting circuits 29a, 29b, 29c, etc., each of which is coupled to each of the electrodes 110a and 110b (with one of the phase shifting circuits remaining unused). The output voltages from these phase-shifting circuits 29a, 29b, 29c have substantially the same amplitudes, however, the phases of the voltages are shifted relative to each other. The phase-shifting circuits 29a, 29b, 29c shift the phases of the voltage line based on the number of electrodes 110a and 110b connected thereto. In other words, the phase shift may be defined by the following formula (1):

$$(1) \qquad \phi = \frac{360°}{n}$$

In formula (1), n is the number of groups of electrodes. More specifically, a group of electrodes may include one or more electrodes. The electrode assembly 100 may include a total of four electrodes, with two groups of two electrodes. In this instance, each of the groups of the electrodes is coupled to one of the two phase-shifting circuits 29a, 29b, 29c. Based on the formula (I), the phase shift between the two groups of electrodes is 180°. In the embodiment of Figs. 3 and 4, where there are two electrodes 110a and 110b, each electrodes forms its own group, therefore the voltages supplied thereto are shifted by 180°.

In use, the generator 20 supplies phased electrosurgical voltage to each of the electrodes 110a and 110b. In other words, the phase-shifting circuits 29a, 29b, 29c energize one of the electrodes 110a and 110b, while the non-energized electrodes 110a and 110b act as a return electrode. This flow of energy between the electrodes 110a and 110b is due to the phase shift therebetween, which provides for a sufficient potential difference therebetween. The electrodes 110a and 110b shift in their roles as active and return electrodes due to the phase-shifted application of voltage.

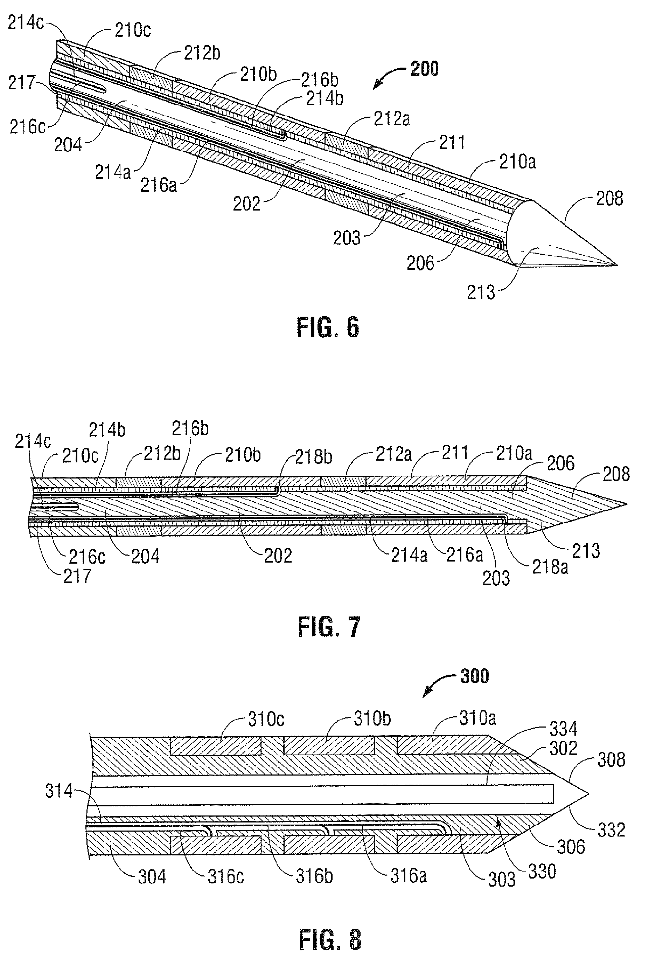

Figs. 6 and 7 show another embodiment of an electrosurgical electrode assembly 200 according to another embodiment of the present disclosure. The electrode assembly 200 includes a core 202 having an elongated cylindrical body portion 203. The body portion 203 includes a proximal end 204 coupled to the housing 6 (Fig. 1) and a distal end 206.

The electrode assembly 200 also includes three or more electrodes 210a, 210b, 210c. The electrodes 210a, 210b, 210c may be formed from any suitable medical grade conductor suitable for contacting tissue (e.g., stainless steel). The electrodes 210b and 210c have a substantially cylindrical tubular shape and are disposed about the core 202. The electrode 210a includes a proximal portion 211 that also has a substantially cylindrical tubular shape and is disposed about the core 202 and a distal portion 213 that includes a tip 208. The tip 208 includes a tapered portion terminating in a sharp tip to allow for insertion into tissue with minimal resistance. In those cases where the energy applicator is inserted into a pre-existing opening, the tip 208 may be rounded or flat.

The electrodes 210a, 210b, 210c are separated by dielectric spacers 212a and 212b, with the dielectric spacer 212a disposed between the electrodes 210a and 210b and the dielectric spacer 212b disposed between the electrodes 210b and 210c. The dielectric spacers 212a and 212b also have a cylindrical tubular shape. In one embodiment, the dielectric spacers 212a and 212b may be integrally formed with the core 202.

The core 202 includes multiple channels 214a, 214b, 214c defined therein. In this embodiment, the conductors 216a, 216b, 216c include an insulating sheath to prevent short-circuiting. In another embodiment, the channels 214a, 214b, 214c house one or more conductors 216a, 216b, 216c, respectively. The conductors 216a, 216b, 216c may be encased in an insulating sheath (not shown) and are coupled to the electrodes 210a, 210b, 210c, respectively. The channels 214a, 214b, 214c are disposed apart (e.g., radially) within the core 202 and extend up to the respective electrodes 210a, 210b, 210c. The dielectric structure of the core 202 obviates the need for providing an insulating sheath about the conductors 216a, 216b, 216c since the conductors 216a, 216b, 216c are separated by the core 202 and only contact the corresponding electrodes 210a, 210b, 210c.

The channels 214a, 214b, 214c are disposed on an outer surface of the core 202. As shown in Fig. 7, the core 202 may include a sheath 217 disposed over the body portion 213 to insulate the conductors 216a, 216b, 216c. The sheath 217 includes openings 218a, 218b, 218c to provide for coupling of the conductors 216a, 216b, 216c to the electrodes 210a, 210b, 210c.

Each of the electrodes 210a, 210b, 210c is connected to each of a plurality of phase-shifting circuits 29a, 29b, 29c, etc. The output voltages from these phase-shifting circuits 29a, 29b, 29c have substantially the same amplitudes, however, the phases of the voltages are shifted relative to each other. In one embodiment, each of electrodes 210a, 210b, 210c forms its own group, therefore the voltages supplied thereto are also shifted by 120° based on formula (1). In use, as each of the electrodes 210a, 210b, 210c is energized by the phase-shifting circuits 29a, 29b, 29c, the remaining two electrodes 210a, 210b, 210c that are not energized act as a return electrode. In other words, the electrodes 210a, 210b, 210c shift roles as active and return electrodes due to the phase-shifted application of voltage. In another embodiment, two of the electrodes (e.g., electrodes 210a and 210c) form one group and the remaining electrode (e.g., electrode 210b) forms another group, hence, the voltages supplied to the two groups are shifted by 180°.

Fig. 8 shows an electrosurgical electrode assembly 300, not falling within the scope of the independent claim below that is substantially similar to the electrode assembly 200. The electrode assembly 300 includes a core 302 having an elongated cylindrical body portion 303. The body portion 303 includes a proximal end 304 coupled to the housing 6 (Fig. 1) and a distal end 306.

The body portion 303 also includes a lumen 330 defined therethrough and the electrode assembly 300 includes an outer tube 332 and an inner tube 334 disposed therein. The outer and inner tubes 332 and 334 are concentrically-disposed relative to each other and are coupled to the coolant system 15 allowing the coolant system 15 to circulate the coolant fluid 19 therethrough. The outer tube 332 includes a tip 308 having a tapered portion terminating in a sharp tip to allow for insertion into tissue with minimal resistance. In those cases where the energy applicator is inserted into a pre-existing opening, the tip 308 may be rounded or flat. In another embodiment the lumen 330 may not pass through the entire body portion 303 and may terminate at the distal end 306 of the core 302, in which case the tip 308 may be formed by the core 302 and/or an electrode 310a as discussed above.

The electrode assembly 300 also includes three or more electrodes 310a, 310b, 310c. The electrodes 310a, 310b, 310c have a substantially cylindrical tubular shape and are disposed about the core 302. The electrode 310a may be tapered to provide continuity for the tip 308. The electrodes 310a, 310b, 310c are separated by dielectric spacers 312a and 312b that are integrally formed with the core 302.

The core 302 includes a channel 314 defined therein for routing the plurality of conductors 316a, 316b, 316c to the electrodes 310a, 310b, 310c. In this embodiment, the conductors 316a, 316b, 316c include an insulating sheath to prevent short-circuiting and are coupled to the electrodes 310a, 310b, 310c, respectively. Each of the electrodes 310a, 310b, 310c is connected to each of a plurality of phase-shifting circuits 29a, 29b, 29c, etc. as discussed above with respect to the electrode assembly 200.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

**Claims**

1.  An electrosurgical instrument (100, 200), comprising:

    a core (102, 202) including an elongated body portion (103, 203), the core including a plurality of channels (114a, 114b, 214a, 214b) defined therein that are disposed radially apart ;
    a plurality of electrodes (110a, b, c; 210 a, b, c) disposed about the core, wherein each of the plurality of electrodes has a tubular shape , the electrodes being spaced apart by one or more dielectric spacers (112, 212a, 212b), the plurality of the electrodes being couplable to an electrosurgical generator (20) configured to supply phased electrosurgical voltage to the plurality of electrodes to generate a potential difference between at least two of the plurality of the electrodes; and
    a plurality of conductors (116a, 116b, 216a), configured to couple respective ones of the plurality of electrodes to the electrosurgical generator, each of the plurality of conductors being disposed within a respective one of the plurality of channels;
    **characterised in that** the plurality of channels are defined on an outer surface of the core (102, 202) and **in that** the plurality of conductors are each formed from a spring member.

2.  An electrosurgical instrument according to claim 1, wherein the core includes a tapered tip (108, 208) at a distal end thereof configured to penetrate tissue.

3.  An electrosurgical instrument according to any one of the preceding claims, wherein a distal most electrode (310a) of the plurality of electrodes includes a tapered tip at a distal end thereof configured to penetrate tissue.

4.  An electrosurgical instrument according to any one of the preceding claims, wherein the core includes a lumen (330) defined therethrough.

5.  An electrosurgical instrument according to claim 4, further comprising:

    an outer tube (332) disposed within the lumen; and
    an inner tube (334) disposed concentrically relative to the outer tube, wherein the outer tube and the inner tube are coupled to a coolant system configured to circulate a coolant therethrough.

6.  An electro surgical instrument according to claim 1, wherein the at least one dielectric spacer is formed integrally with the core.

7.  An electrosurgical instrument according to claim 1, wherein the conductors are separated by the core and only contact the corresponding electrodes, and wherein the core has a dielectric structure so that the dielectric structure of the core obviates the need for an insulating sheath about each conductor.

8.  An electrosurgical system (1), comprising:

    an electrosurgical instrument (100, 200) according claim 1; and
    an electrosurgical generator (20) coupled to each of the plurality of the electrodes and configured to supply phased electrosurgical voltage to the plurality of electrodes to generate a potential difference between at least two of the plurality of the electrodes.

9.  An electrosurgical system according to claim 1, further comprising:

    a coolant system (15) including:

        a supply tank (18) configured to store a coolant (19); and
        a supply pump (16) coupled to the supply tank and to the electrosurgical instrument and configured to circulate a coolant therethrough.

**Patentansprüche**

1.  Ein Operationsinstrument (100, 200), umfassend:

    einen Kern (102, 202) mit einem langgestreckten Körperabschnitt (103, 203), wobei der Kern eine Vielzahl an darin definierten und radial getrennt angeordneten Kanälen (114a, 114b, 214a, 214b) aufweist;

eine Vielzahl an Elektroden (110a, b, c; 210a, b, c), über dem Kern angeordnet, wobei die Vielzahl an Elektroden röhrenförmig ist, die Elektroden durch einen oder mehrere dielektrische Abstandhalter (112, 212a, 212b) räumlich getrennt sind, die Vielzahl an Elektroden mit einem elektrochirurgischen Generator (20) kuppelbar sind, der konfiguriert ist, um eine elektrochirurgische Phasenspannung an die Vielzahl an Elektroden zu liefern, um zwischen mindestens zwei der Vielzahl an Elektroden einen Potentialdifferenz zu generieren; und

eine Vielzahl an Leitern (116a, 116b, 216a), die konfiguriert sind, um die einzelnen Elektroden aus der Vielzahl an Elektroden mit dem elektrochirurgischen Generator zu kuppeln, wobei jeder Leiter der Vielzahl an Leitern in einem entsprechenden Kanal der Vielzahl an Kanälen angeordnet ist;

**dadurch gekennzeichnet, dass** die Vielzahl an Kanälen an einer Außenfläche des Kerns (102, 202) definiert ist und dass jeder Leiter der Vielzahl an Leitern von einem Federglied gebildet wird.

2. Ein Operationsinstrument gemäß Anspruch 1, **dadurch gekennzeichnet, dass** der Kern an einem distalen Ende eine verjüngte Spitze (108, 208) enthält, die konfiguriert ist, um Gewebe zu durchdringen.

3. Ein Operationsinstrument gemäß einem der vorstehenden Ansprüche, **dadurch gekennzeichnet, dass** eine distalste Elektrode (310a) der Vielzahl an Elektroden an einem distalen Ende eine verjüngte Spitze enthält, die konfiguriert ist, um Gewebe zu durchdringen.

4. Ein Operationsinstrument gemäß einem der vorstehenden Ansprüche, **dadurch gekennzeichnet, dass** der Kern ein hindurch verlaufendes Lumen (330) enthält.

5. Ein Operationsinstrument gemäß Anspruch 4, **dadurch gekennzeichnet, dass** es

ein äußeres Rohr (332), das im Lumen angeordnet ist; und

ein inneres Rohr (334), das in Bezug auf das äußere Rohr konzentrisch angeordnet ist, umfasst, wobei das äußere und das innere Rohr mit einem Kühlsystem gekuppelt sind, welches konfiguriert ist, um ein Kühlmittel hindurch zu pumpen.

6. Ein Operationsinstrument gemäß Anspruch 1, **dadurch gekennzeichnet, dass** mindestens ein dielektrischer Abstandhalter einstückig mit dem Kern gebildet ist.

7. Ein Operationsinstrument gemäß Anspruch 1, **dadurch gekennzeichnet, dass** die Leiter durch den Kern getrennt sind und nur die entsprechenden Elektroden berühren, und dass der Kern eine dielektrische Struktur aufweist, sodass die dielektrische Struktur den Bedarf an einer isolierenden Hülle über jedem Leiter überflüssig macht.

8. Ein elektrochirurgisches Operationssystem (1), umfassend:

ein Operationsinstrument (100, 200) gemäß Anspruch 1; und

einen elektrochirurgischen Generator (20), der an jede der Vielzahl an Elektroden gekuppelt und konfiguriert ist, um eine elektrochirurgische Phasenspannung an die Vielzahl an Elektroden zu liefern, um zwischen mindestens zwei der Vielzahl an Elektroden einen Potentialdifferenz zu generieren.

9. Ein elektrochirurgisches Operationssystem gemäß Anspruch 1, das darüber hinaus ein Kühlsystem (15) mit:

einem Vorratsbehälter (18), der zur Aufbewahrung eines Kühlmittels (19) konfiguriert ist; und

einer Versorgungspumpe (16), die an den Vorratsbehälter und das Operationsinstrument gekuppelt ist und konfiguriert ist, um ein Kühlmittel hindurch zu pumpen, umfasst.

## Revendications

1. Instrument électrochirurgical (100, 200), comprenant :

un coeur (102, 202) incluant une partie formant corps allongé (103, 203), le coeur incluant une pluralité de canaux (114a, 114b, 214a, 214b) définis en son sein qui sont disposés espacés de façon radiale ;

une pluralité d'électrodes (110a, b, c ; 210a, b, c) disposées autour du coeur, dans lesquelles chaque électrode de la pluralité d'électrodes a une forme tubulaire, les électrodes étant espacées par une ou plusieurs entretoises diélectriques (112, 212a, 212b), la pluralité des électrodes pouvant être reliée à un générateur électrochirurgical (20) configuré pour fournir une tension électrochirurgicale en phase à la pluralité d'électrodes pour produire une différence de potentiel entre au moins deux électrodes de la pluralité des électrodes ; et

une pluralité de conducteurs (116a, 116b, 216a), configurés pour relier des électrodes respectives de la pluralité d'électrodes au générateur électrochirurgical, chaque conducteur de la pluralité de conducteurs étant disposé à l'intérieur d'un canal respectif de la pluralité de canaux ; **caractérisé en ce que** la pluralité de canaux est définie sur une surface extérieure du coeur (102, 202) et **en ce que** la pluralité de conducteurs est formée, chacun, à partir d'un élément élastique.

2. Instrument électrochirurgical selon la revendication 1, dans lequel le coeur inclut une pointe tronconique (108, 208) au niveau d'une extrémité distale de ce dernier, configurée pour pénétrer dans un tissu.

3. Instrument électrochirurgical selon n'importe laquelle des revendications précédentes, dans lequel une électrode la plus distale (310a) de la pluralité d'électrodes inclut une pointe tronconique au niveau d'une extrémité distale de celle-ci configurée pour pénétrer dans un tissu.

4. Instrument électrochirurgical selon n'importe laquelle des revendications précédentes, dans lequel le coeur inclut une lumière (330) définie à travers ce dernier.

5. Instrument électrochirurgical selon la revendication 4, comprenant en outre :

  un tube extérieur (332) disposé à l'intérieur de la lumière ; et
  un tube intérieur (334) disposé de manière concentrique par rapport au tube extérieur, dans lequel le tube extérieur et le tube intérieur sont reliés à un système à liquide de refroidissement configuré pour faire circuler à travers ce dernier un liquide de refroidissement.

6. Instrument électrochirurgical selon la revendication 1, dans lequel l'au moins une entretoise diélectrique est formée d'un seul tenant avec le coeur.

7. Instrument électrochirurgical selon la revendication 1, dans lequel les conducteurs sont séparés par le coeur et entrent seulement en contact avec les électrodes correspondantes, et dans lequel le coeur a une structure diélectrique de sorte que la structure diélectrique du coeur obvie au besoin d'une gaine isolante autour de chaque conducteur.

8. Système électrochirurgical (1), comprenant :

  un instrument électrochirurgical (100, 200) selon la revendication 1 ; et

  un générateur électrochirurgical (20) relié à chaque électrode de la pluralité des électrodes et configuré pour fournir une tension électrochirurgicale en phase à la pluralité d'électrodes pour produire une différence de potentiel entre au moins deux électrodes de la pluralité des électrodes.

9. Système électrochirurgical selon la revendication 1, comprenant en outre :

  un système à liquide de refroidissement (15) incluant :

    un réservoir d'alimentation (18) configuré pour stocker un liquide de refroidissement (19) ; et
    une pompe d'alimentation (16) reliée au réservoir d'alimentation et à l'instrument électrochirurgical et configurée pour faire circuler un liquide de refroidissement à travers ce dernier.

FIG. 1

**FIG. 2**

FIG. 3

FIG. 4

FIG. 5

**FIG. 6**

**FIG. 7**

**FIG. 8**

**EP 2 322 111 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2001008967 A

- US 5935159 A